(19) Europäisches Patentamt
European Patent Office
Office européen des brevets

(11) Publication number: **0 173 807 A1**

(12) **EUROPEAN PATENT APPLICATION**

(21) Application number: 85107718.0

(22) Date of filing: 21.06.85

(51) Int. Cl.⁴: C 07 D 249/08
A 01 N 43/653

(30) Priority: 22.06.84 JP 128486/84

(43) Date of publication of application: 12.03.86 Bulletin 86/11

(84) Designated Contracting States: CH DE FR GB IT LI NL

(71) Applicant: MITSUBISHI CHEMICAL INDUSTRIES LIMITED
5-2, Marunouchi 2-chome Chiyoda-ku
Tokyo 100(JP)

(72) Inventor: Yoshida, Kenji
1-18-52, Misono
Sagamihara-shi Kanagawa-ken(JP)

(72) Inventor: Ochiai, Rie
3-3-30, Inamuragasaki
Kamakura-shi Kanagawa-ken(JP)

(72) Inventor: Nakajima, Tetsuo
5-6, Tsutsujigaoka Midori-ku
Yokohama-shi Kanagawa-ken(JP)

(72) Inventor: Tsuda, Masataka
2-5-8, Ogawa
Machida-shi Tokyo(JP)

(74) Representative: Vossius Vossius Tauchner Heunemann Rauh
Siebertstrasse 4 P.O. Box 86 07 67
D-8000 München 86(DE)

(54) **Triazolylacyloxy ethanes, fungicide containing the same as an active ingredient and method of controlling fungi using the same.**

(57) This invention relates to novel triazolylacyloxy ethanes represented by the following general formula:

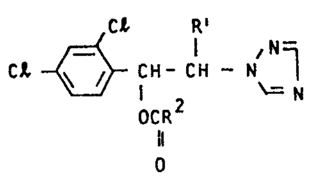

where $R^1$ represents linear alkyl; linear or branched alkenyl; or linear or branched alkynyl; $R^2$ represents linear or branched alkyl optionally substituted with halogen; or linear or branched alkenyl; cycloalkyl; phenyl optionally substituted with lower alkyl or halogen; or lower alkoxy, and to a fungicide containing the above mentioned compounds as an active ingredient and a method of controlling fungi by using the same.

EP 0 173 807 A1

VOSSIUS · VOSSIUS · TAUCHNER
HEUNEMANN · RAUH
PATENTANWÄLTE
SIEBERTSTR. 4, 8000 MÜNCHEN 80
TEL. (089) 47 40 75

0172807

Our Ref: T 827 EP
Mitsubishi Chemical Industries,
Limited
Tokyo/Japan
Case: F-3465

June 21, 1985

# TRIAZOLYLACYLOXY ETHANES, FUNGICIDE CONTAINING THE SAME AS AN ACTIVE INGREDIENT AND METHOD OF CONTROLLING FUNGI USING THE SAME

This invention concerns triazolylacyloxy ethanes. More specifically it relates to novel 1-(2,4-dichlorophenyl)-1-acyloxy-2-(1H-1,2,4-triazol-1-yl) ethanes, a fungicide containing the above-mentioned compounds as an active ingredient and a method of controlling fungi by using the same.

It has been known so far that certain kind of triazolyl ethane compounds have fungicidal activities.

For instance, US Patent No. 4315016 describes that the compound represented by the following general formula (A) has a fungicidal activity:

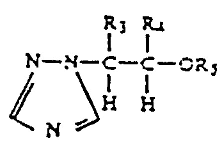

(A)

wherein $R_3$ is hydrogen, $R_4$ is phenyl optionally substituted with one or two halogen, and $R_5$ is alkyl

having up to 5 carbon atoms, alkenyl having up to 5 carbon atoms, alkynyl having up to 5 carbon atoms or $R_5$ is benzyl optionally ring substituted with halogen, alkyl or alkoxy, or a fungicidal acid salt of such a compound.

Further, US Patent No. 4349556 describes that the compound represented by the following general formula (B) has fungicidal and nematicidal activities.

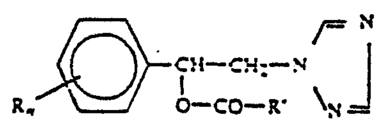
(B)

in which

R is halogen; alkyl with 1 to 4 carbon atoms; alkoxy with 1 or 2 carbon atoms; alkylthio with 1 or 2 carbon atoms; alkyl sulfonyl with 1 to 4 carbon atoms; haloalkyl with up to 4 carbon atoms; nitro; cyano; phenyl; phenoxy; or phenyl or phenoxy substituted by fluorine, chlorine, bromine, cyano, nitro or haloalkyl with 1 or 2 carbon atoms and up to 3 fluorine plus chlorine atoms;

R' is alkyl with 1 to 6 carbon atoms; alkenyl with 2 to 4 carbon atoms; alkynyl with 2 to 4 carbon atoms; cycloalkyl with 5 to 7 carbon atoms; haloalkyl with 1 or

2 carbon atoms; phenyl; phenylalkyl or phenoxyalkyl each with 1 or 2 carbon atoms in the alkyl moiety; such phenyl, phenylalkyl or phenoxyalkyl substituted on the phenyl by halogen, amino, cyano, nitro or alkyl with 1 or 2 carbon atoms; amino; alkylamino, dialkylamino or alkylalkyl-carbonylamino with 1 or 2 carbon atoms in each alkyl moiety; phenylamino; or phenylamino substituted by halogen, nitro or cyano; and

n represents 0, 1, 2, 3, 4 or 5.

Further, DE Patent 2556319 describes that the compound represented by the following general formula (C) has a fungicidal activity:

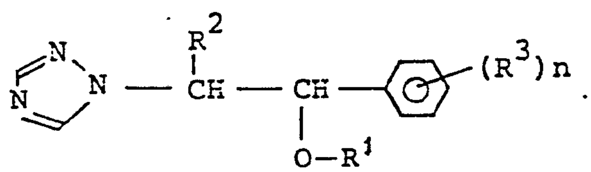

(C)

in which

$R^1$ is alkyl with 1 to 8 carbon atoms optionally substituted with 1 or several halogen, alkoxy or cyano, alkenyl with 3 to 6 carbon atoms optionally substituted with halogen, alkynyl with 3 to 6 carbon atoms optionally substituted with halogen, or benzyl group of the general formula 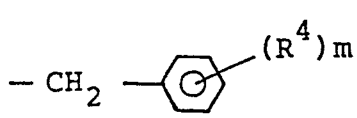 wherein m is

0 to 5, and $R^4$ is alkyl with 1 to 4 carbon atoms;

$R^2$ is hydrogen, alkyl with 1 to 5 carbon atoms, phenyl or cyclohexyl,

n is 0 to 3, and

$R^3$ is fluorine, chlorine, bromine with 1 to 3 carbon atoms.

Furthermore, DE Patent No. 3233145 describes that the compound represented by the following general formula (D) has a fungicidal activity:

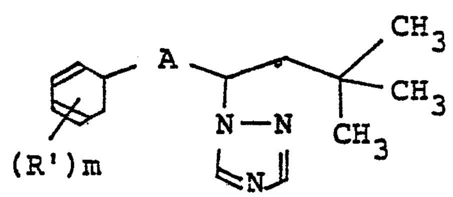

(D)

in which R' is alkyl, phenyl, trifluoromethyl, alkoxy or halogen; m is 0, 1, 2 or 3; A is C=X, CHY or $CR^2R^3$ where X is O or $NOR^4$, Y is $OR^5$, OAcyl or $OSi(CH_3)_3$; $R^2$ is OH or $OCH_3$; $R^3$ is alkyl, aralkyl, aryl or vinyl where $R^4$ and $R^5$ represent individually H, alkyl, allyl, propargyl or aralkyl.

However, the fungicidal activities of these known triazolyl ethanes are not always satisfactory, and such activity may vary or even disappear depending on the presence or absence or the kind of substituents at 1- and

2-positions of ethane, for example, in the case of 1-phenyl-2-(1H-1,2,4-triazol-1-yl) ethanes and it has been quite unknown what compounds have substantial fungicidal activity.

The present inventors have made an earnest study on triazolyl ethanes and, in particular, triazolylacyloxy ethanes and, as a result, have found that the novel compounds according to this invention have an excellent fungicidal activity as compared with known fungicides.

This invention provides triazolylacyloxy ethanes represented by the general formula (I):

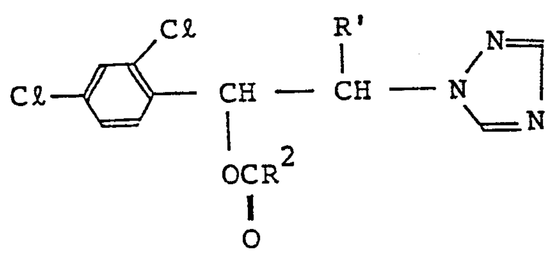

(I)

wherein $R^1$ represents linear alkyl; linear or branched alkenyl; or linear or branched alkynyl; $R^2$ represents linear or branched alkyl optionally substituted with halogen; linear or branched alkenyl; cycloalkyl; phenyl

optionally substituted with lower alkyl or halogen; or lower alkoxy group, as well as a fungicide containing the above-mentioned compounds as an active ingredient and a method of controlling fungi by using the same.

The triazolylacyloxy ethanes according to this invention are novel compoundsrepresented by the general formula (I) as described above.

In the general formula (I), $R^1$ represents linear alkyl; linear or branched alkenyl; or linear or branched alkynyl. Preferably, $R^1$ represents linear alkyl with 1 to 12 carbon atoms such as methyl, ethyl, n-butyl, n-pentyl, n-heptyl, n-octyl, n-nonyl, n-decyl and n-dodecyl; linear or branched alkenyl with 2 to 6 carbon atoms such as allyl, methallyl, butenyl, isobutenyl and isopentenyl; or linear or branched alkynyl with 2 to 6 carbon atoms such as propargyl and butynyl. More preferably, $R^1$ represents linear alkyl with 1 to 10 carbon atoms; linear or branched alkenyl with 2 to 5 carbon atoms or linear alkynyl with 2 to 4 carbon atoms.

In view of the fungicidal activity, $R^1$ preferably represents linear alkyl with 1 to 10 carbon

atoms; linear or branched alkenyl with 3 to 5 carbon atoms; or propargyl and, more preferably, linear alkyl with 1 to 8 carbon atoms.

While on the other hand, $R^2$ in the general formula (I) represents linear or branched alkyl optionally substituted with halogen such as fluorine, chlorine, bromine and iodine; linear or branched alkenyl; cycloalkyl; phenyl optionally substituted with lower alkyl or halogen such as fluorine, chlorine, bromine and iodine; or lower alkoxy. Preferably, $R^2$ represents linear or branched alkyl with 1 to 12 carbon atoms optionally substituted with fluorine, chlorine or bromine such as methyl, ethyl, n-propyl, n-butyl, iso-butyl, n-pentyl, neopentyl, n-hexyl, n-octyl, n-decyl, n-dodecyl, iodomethyl, trifluoromethyl, ω-chloropropyl, α,α-dichloroethyl, β,β-dichloroethyl, α-chloro-β-bromoethyl and α-bromo-n-pentyl; linear or branched alkenyls with 2 to 6 carbon atoms such as allyl, methallyl, butenyl, isobutenyl and isopentenyl; cycloalkyl with 3 to 6 carbon atoms such as cyclopropyl, cyclopentyl and cyclohexyl; phenyl optionally substituted with alkyl with 1 to 3 carbon atoms, fluorine, chlorine or bromine such as phenyl, 4-methylphenyl, 4-chlorophenyl and 2,4-dichlorophenyl; or alkoxy with 1 to 4 carbon atoms such as

methoxy, ethoxy and n-propoxy. In the case where $R^2$ is an alkyl substituted with fluorine, chlorine or bromine, the number of the substituents is 1,2 or more and preferably up to 5 and the substituents preferably occupy α, β and/or ω position. In the case where there are two or more substituents, they may be identical or different with each other. Further, in the case where $R^2$ is a phenyl substituted with alkyl, fluorine, chlorine or bromine, the number of the substituents is 1,2 or more and, preferably up to 3. In the case where there are two or more substituents, they may be identical or different with each other. In view of the fungicidal activity, $R^2$ represents preferably linear or branched alkyl with 1 to 10 carbon atoms optionally substituted with fluorine, chlorine or bromine; or linear or branched alkenyl with 2 to 6 carbon atoms. More preferably, $R^2$ represents linear or branched alkyl with 1 to 10 carbon atoms optionally substituted with fluorine, chlorine or bromine; or alkenyl with 2 or 3 carbon atoms and, further preferably, linear alkyl with 1 to 8 carbon atoms optionally substituted with fluorine or bromine. As the agricultural fungicide, preferred are those compounds represented by the general formula (I) in which $R^1$ is linear alkyl with 1 to 6 carbon atoms and $R^2$ is linear

alkyl with 1 to 8 carbon atoms optionally substituted with chlorine or bromine, and most preferred are those compounds, in which $R^1$ is linear alkyl with 2 to 4 carbon atoms and $R^2$ is linear alkyl with 1 to 6 carbon atoms optionally substituted with chlorine or bromine.

The compounds according to this invention represented by the general formula (I) described above are novel compounds and can be prepared, for instance, by reacting 2-substituted-2-(1H-1,2,4-triazol-1-yl)-1-(2,4-dichlorophenyl) ethanol represented by the following general formula (II) and an acid anhydride represented by the following general formula (III) or an acid halide represented by the following general formula (IV) in the presence of a base:

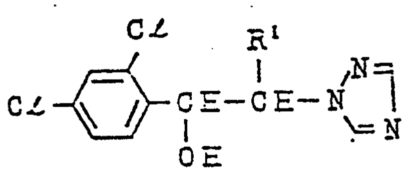

(II)

+ $(R^2CO)_2O$

(III)

or

$R^2COX$

(IV)

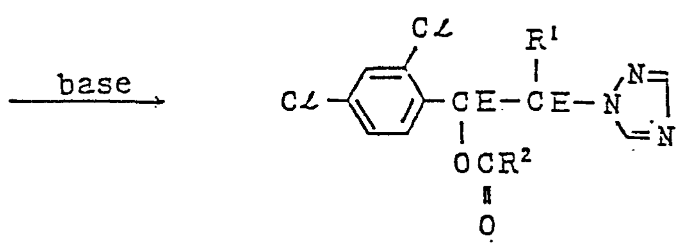


(I)

wherein $R^1$ and $R^2$ have the same meanings as defined in the general formula (I), and X represents halogen such as chlorine or bromine.

The above-mentioned reaction may be carried out in the presence of an inorganic base such as potassium carbonate, sodium carbonate, sodium hydroxide and sodium hydride or an organic base such as pyridine or triethylamine, in an organic solvent such as tetrahydrofuran, benzene, toluene, dimethylformamide and dimethyl sulfoxide or the mixture of these solvents. If the acid anhydride (III) is used as the starting material or if the organic base as mentioned above is used as the base, the same anhydride or base may be used also as the solvent. The reaction temperature usually ranges from -20°C to 120°C, and a suitable temperature range is from 30°C to 100°C in the case of using the acid anhydride (III) or from -5°C to 40°C in the case of using the acid halide (IV) respectively as the starting material. The reaction time is from 0.5 to 20 hours.

The compounds according to this invention thus obtained contain two or more asymmetric carbon atoms and, accordingly, are generally obtained as a mixture of various isomers. In the case where the compounds according to this invention are used as a fungicide, the

isomer mixture may be used as it is, or it can be used after being separated into each of the isomers by way of various known methods.

The intermediate represented by the general formula (II) as described above can be prepared, for example, in accordance with descriptions in British Patent Nos. 1529818 and 1544028 by treating α-substituted -α-(1H-1,2,4-triazol-1-yl) acetophenone or 1-substituted -2-(2,4-dichlorobenzoyl)-2-(1H-1,2,4-triazol-1-yl) ethene with a reducing agent such as sodium borohydride.

The compounds according to this invention obtained in this way have an excellent fungicidal activity. Particularly, they have an excellent protective effect against pathogenic fungi for various kinds of plants and is useful as the agricultural fungicide.

In the case of using the compounds according to this invention as an agricultural fungicide, the compounds may be used as they are but, preferably, in the form of emulsifiable concentrate, wettable powder, dust, granule or the like by adding adjuvants in the conventional manner for the effective dispersion of the active ingredient in the application site.

The adjuvants for use with the agricultural fungicide according to this invention can include

solvent, carrier, wetting agent, dispersing agent, emulsifier, penetrating agent, which are used properly depending on the requirement. Suitable solvent can include, for example, water; alcohols such as methanol, ethanol or ethylene glycol; ketones such as acetone, methyl ethyl ketone or cyclohexanone; ethers such as ethyl ether, dioxane or cellosolves; aliphatic hydrocarbons such as kerosene, light oil or fuel oils; aromatic hydrocarbons such as benzene, toluene, xylene, solvent naphtha or methyl naphthalene; halogenated hydrocarbons such as dichloroethane, trichlorobenzene or carbon tetrachloride; acid amides such as dimethylform amide; esters such as ethyl acetate, butyl acetate or glycerine ester of fatty acid; nitriles such as acetonitrile and they may be used alone or in admixture.

Suitable carrier can include mineral powder, for example, clays such as kaolin or bentonite; talcs such as talc or pyrophylite; oxides such as diatomaceous earth or white carbons; and plant powder such as soybean powder or CMC, which may be used alone or in admixture.

Further, surface active agent may be used as the wetting agent, dispersing agent, emulsifier and penetrating agent. The surface active agent can include, for example, non-ionic surfactant such as

polyoxyethylenealkylaryl ether, polyoxyethylenesorbitan monolaurate, etc.; cationic surfactant such as alkyldimethylbenzylammonium chloride, alkylpyridinium chloride, etc.; anionic surfactant such as alkylbenzene sulfonate, lignin sulfonate or higher alcohol sulfate; amphoteric surfactant such as alkyldimethylbetaine, dodecylaminoethyl glycine, etc.

These surface active agents may be used alone or in admixture depending on the application uses.

In the case where the agricultural fungicide according to this invention is used in the form of an emulsifiable concentrate, a formulated concentrate is prepared by mixing from 10 to 50 parts of the compound according to this invention, from 10 to 80 parts of a solvent and from 3 to 20 part of a surface active agent in an appropriate ratio, which is diluted with water to a prescribed concentration upon use.

Further, in the case of use as a wettable powder, from 5 to 80 parts of the compound according to this invention are mixed with from 10 to 90 parts of a carrier and from 1 to 20 parts of a surface active agent in an appropriate ratio and the mixture is diluted in the same manner as in the case of the emulsifiable concentrate with water or the like upon use.

In the case of use as a dust, from 0.1 to 5 parts of the compound according to this invention are usually mixed uniformly with from 95 to 99.9 parts of a carrier such as kaolin, bentonite or talc for use.

Further in the case of use as a granule, from 0.1 to 15 parts of the compound according to this invention are usually mixed with from 82 to 98 parts of a carrier such as bentonite, clay or talc and from 1 to 3 parts of a surface active agent in an appropriate ratio, which is then pelletized for use.

The agricultural fungicide containing the compound according to this invention as the active ingredient usually contains from 0.1 to 80% by weight of the compound according to this invention and from 20 to 99.9% by weight of an inert other component irrespective of the mode of the application.

Further, the agricultural fungicide according to this invention may be used in admixture with other active ingredient that will not hinder the fungicidal activity of the active ingredient, for example, ordinary fungicide, insecticide and acaricide.

Referring to the method of application, the agricultural fungicide according to this invention may be suitably applied in either of the foliar application

or submerged application. In the case of the foliar application, usually, a dust containing from 0.1 to 5% by weight of the active ingredient may be applied in an amount from 2 to 6 kg per 10 ares, or an emulsifiable concentrate or wettable powder diluted with water so as to contain from 10 to 1000 ppm of the active ingredient may be used in an amount from 10 to 500 liter per 10 ares. In the case of the submerged application, it may be applied in the form of granule containing from 0.1 to 15% by weight of the active ingredient in an amount from 2 to 6 kg per 10 ares.

Any of the compounds according to this invention has an excellent fungicidal activity and has an intense fungicidal activity, particularly, as compared with known triazole compound, against wide variety of plant diseases, for example, rice blast, grey mold of various crops, various rust of wheat or barley, and powdery mildew of various crops. It has a particularly strong fungicidal activity to the rice blast and powdery mildew of wheat or barley and is useful as a non-medical fungicide, particularly, agricultural fungicide.

Further, since the compound according to this invention is scarcely harmful to plants although it has a systemic activity to plants, and less toxic to men and

beast or fishes, it is very useful for the control of plant diseases.

Furthermore, the compounds according to this invention, when used to the rice patty field in the submerged application, have an effect of suppressing the growth of rice plant with no undesired effect for the yield and it is useful also as an antilodging agent.

This invention will now be described more specifically referring to preparation examples of the compound according to this invention, formulation examples of the fungicides containing the above-mentioned compound and protective test examples and growth suppressing test examples using the fungicide according to this invention. It should be noted that this invention is no way restricted to these specifically illustrated examples. In the specification hereinafter "parts" mean "parts by weight".

The chemical structures of the compounds according to this invention synthesized in the preparation examples are identified by elementary analysis, IR spectrum, NMR spectrum, etc.

Preparation Example 1 (Preparation of Intermediate-1):

To 100 mℓ of methanol, were dissolved 7.6 g of α-(1H-1,2,4-triazol-1-yl)-2,4-dichloropropiophenone, into which 1.3 g of sodium borohydride were added under ice cooling. After stirring at room temperature for three hours to complete the reaction, the solvent was distilled off under a reduced pressure. Ethyl acetate was added to the residue and, after washing with water, the solvent was removed by distillation. The residue was purified by silica gel column chromatography eluted with a chloroform - ethyl acetate mixture to obtain 5.9 g of a diastereomer mixture of 1-(2,4-dichlorophenyl)-2-(1H-1,2,4-triazol-1-yl) propan-1-ol (compound No. 1 in Table 1). In the similar manner, compounds No. 2, No. 3 and No. 6 shown in Table 1 were obtained.

Preparation Example 2 (Preparation of Intermediate-2):

To 50 mℓ of anhydrous dimethylformamide, were dissolved 12.8 g of α-(1H-1,2,4-triazol-1-yl)-2,4 -dichloroacetophenone, into which 2.4 g of 50% sodium borohydride were added. After the completion of hydrogen generation, 6.05 g of allyl bromide were added dropwise at room temperature. Stirring was continued at that temperature for two hours to complete the reaction.

After adding ethyl acetate to the reaction product and washing with water, the solvent was distilled off under a reduced pressure and the residue was purified by silica gel chromatography eluted with a dichloromethane-diethyl ether mixture to obtain 4.3 g of α-allyl-α-(1H-1,2,4-triazol-1-yl)-2,4-dichloroacetophenone as an intermediate.

The similar reaction as in the Preparation Example 1 was carried out except for using the thus obtained α-allyl-α-(1H-1,2,4-triazol-1-yl)-2,4-dichloroacetophenone instead of α-(1H-1,2,4-triazol-1-yl)-2,4-diochloropropiophenone, to obtain a diastereomer mixture of 1-(2,4-dichlorophenyl)-2-(1H-1,2,4-triazol-1-yl)-4-penten-1-ol (compound No. 4 in Table 1).

In the similar manner, compounds No. 5, No. 7, No. 8 and No. 10 shown in Table 1 were obtained.

Preparation Example 3 (Preparation of Intermediate-3):

To 300 mℓ of toluene, were added 20.5 g of α-(1H-1,2,4-triazol-1-yl)-2,4-dichloroacetophenone, 15.0 g of propionic aldehyde, 0.68 g of pyperazine and 0.96 g of acetic acid, and they were reacted for two hours under reflux while removing resulting water. After cooling, the reaction mixture was washed with water and the

solvent was distilled off under a reduced pressure. The residue was purified by silica gel column chromatography eluted with a chloroform - ethyl acetate mixture to obtain 20.1 g of 1-(2,4-dichlorophenyl)-2-(1H-1,2,4-triazol-1-yl)-pent-2-en-1-on.

18.1 g of the obtained 1-(2,4-dichlorophenyl)-2-(1H-1,2,4-triazol-2-yl)-pent-2-en-1-on were dissolved in 200 mℓ of methanol, into which 4.6 g of sodium borohydride were added under ice cooling. After stirring at room temperature for three hours to complete the reaction, the solvent was distilled off under a reduced pressure. The residue was purified by silica gel column chromatography eluted with a chloroform - ethyl acetate mixture to obtain 9.9 g of diastereomer mixture of 1-(2,4-dichlorophenyl)-2-(1H-1,2,4-triazol-1-yl)-penthan-1-ol (compound No. 3 in Table 1). This was quite the same compound as the compound No. 3 obtained in Preparation Example 1.

In the similar manner, the compound No. 9, No. 11 and No. 12 shown in Table 1 were obtained.

Table 1

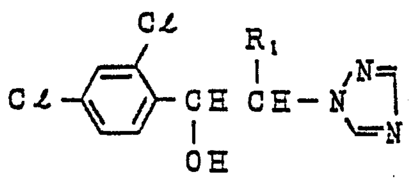

| Compound No. | $R^1$ | Isomer *1 | Melting point or refractive index | |
|---|---|---|---|---|
| 1 | $CH_3$ - | M | $n_D^{25}$ | 1.5669 |
| 2 | $C_2H_5$ - | M | mp | 113-3.5°C |
| 3 | n - $C_3H_7$ - | M | $n_D^{25}$ | 1.5515 |
| 4 | $CH_2 = CHCH_2$ - | M | $n_D^{25}$ | 1.5642 |
| 5 | $CH \equiv CCH_2$ - | M | $n_D^{25}$ | 1.5508 |
| 6 | n - $C_4H_9$ - | M | $n_D^{25}$ | 1.5484 |
| 7 | $CH_3CH = CHCH_2$ - | M | $n_D^{25}$ | 1.5652 |
| 8 | $CH_2 = C(CH_3)CH_2$ - | M | $n_D^{25}$ | 1.5630 |
| 9 | n - $C_5H_{11}$ - | M | $n_D^{25}$ | 1.5393 |
| 10 | $(CH_3)_2C = CHCH_2$ - | M | $n_D^{25}$ | 1.5670 |
| 11 | n - $C_7H_{15}$ - | M | $n_D^{25}$ | 1.5308 |
| 12 | n - $C_{10}H_{21}$ - | M | $n_D^{25}$ | 1.5266 |

*1 : M indicates that the isomer is a diastereomer mixture.

Preparation Example 4:

A mixture of 2.72 g of the compound No. 1 in Table 1, 3.0 g of potassium carbonate and 20 mℓ of anhydrous acetic acid was stirred at 60°C for three hours. After cooling, ethyl acetate was added to the reaction mixture and, after washing with water, the solvent and the excess acetic anhydride were distilled off under a reduced pressure. The residue was purified by silica gel column chromatography eluted with a chloroform - ethyl acetate mixture to obtain 2.6 g of diastereomer mixture of 1-(2,4-dichlorophenyl)-1-acetyloxy -2-(1H-1,2,4-triazol-1-yl)propane (compound No. 13 in Table 2).

In the similar manner, the compounds No. 14, No. 41 and No. 44 shown in Table 2 were obtained.

Preparation Example 5:

Reaction and purification were carried out in the similar manner as in Preparation Example 4 except for using 3.52 g of the compound No. 3 in Table 1 to obtain 1.22 g of the isomer A (compound No. 30 in Table 2) and 2.40 g of the isomer B (compound No. 31 in Table 2) of 1-(2,4-dichlorophenyl)-1-acetyloxy-2-(1H-1,2,4-triazol-1 -yl) pentane.

Preparation Example 6:

To 20 mℓ of anhydrous dimethylformamide, 2.00 g of the compound No. 2 shown in Table 1 were dissolved, to which 0.50 g of 50% sodium hydride were added. After the completion of hydrogen generation, 0.97 g of propionic chloride were added dropwise. After the dropping was over, it was stirred at room temperature for two hours to complete the reaction. After adding ethyl acetate and washing with water, the solvent was distilled off under a reduced pressure and the residue was purified by silica gel column chromatography eluted with a chloroform - ethyl acetate mixture to obtain 1.7 g of a diastereomer mixture of 1-(2,4-dichlorophenyl)-1-propionyloxy-2-(1H -1,2,4-triazol-1-yl) butane (compound No. 16 in Table 2).

In the similar manner, compounds Nos. 15, 19, 20, 23, 24, 27, 32, 33, 34, 35, 36, 37, 38, 39, 40, 42, 43, 45, 46, 47, 48, 49, 52, 53, 54, 55, 56, 57, 58, 59, 60, 61 and 64 shown in Table 2 were obtained.

Preparation Example 7:

Using the compound No. 2 shown in Table 1 and benzoyl chloride, the reaction and purification were carried out in the similar manner as in Preparation Example 6 to obtain the isomer A (compound No. 25 in

Table 2) and the isomer B (compound No. 26 in Table 2) of 1-(2,4-dichlorophenyl)-1-benzoyloxy-2-(1H-1,2,4-triazol-1-yl) butane.

In the similar manner, the compounds Nos. 17, 18, 21, 22, 28, 29, 50, 56, 62, 63, 65, 66, 67 and 68 shown in Table 2 were obtained.

Examples of the compounds according to this invention prepared in this way are shown in Table 2.

Table 2

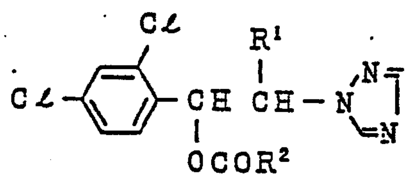

| Compound No. | $R^1$ | $R^2$ | Isomer* | Physical property | |
|---|---|---|---|---|---|
| 13 | $CH_3$- | $CH_3$- | M | $n_D^{25}$ | 1.5413 |
| 14 | $C_2H_5$- | $CH_3$- | M | mp | 113 - 3.5°C |
| 15 | $C_2H_5$- | $CF_3$- | M | $n_D^{25}$ | 1.5008 |
| 16 | $C_2H_5$- | $C_2H_5$- | M | mp | 72 - 3°C |
| 17 | $C_2H_5$- | $CH_2$=CH- | A | mp | 84.7 - 5.6°C |
| 18 | $C_2H_5$- | $CH_2$=CH- | B | mp | 127 - 7.7°C |
| 19 | $C_2H_5$- | n-$C_3H_7$- | M | $n_D^{25}$ | 1.5272 |
| 20 | $C_2H_5$- | i-$C_3H_7$- | M | | semi-solid |
| 21 | $C_2H_5$- | cyc-$C_3H_5$- | A | $n_D^{25}$ | 1.5374 |
| 22 | $C_2H_5$- | cyc-$C_3H_5$- | B | mp | 123 - 3.5°C |
| 23 | $C_2H_5$- | n-$C_5H_{11}$- | M | $n_D^{25}$ | 1.5209 |
| 24 | $C_2H_5$- | cyc-$C_6H_{11}$- | M | $n_D^{25}$ | 1.5390 |
| 25 | $C_2H_5$- | Ph-** | A | mp | 116 - 7°C |

| Compound No. | $R^1$ | $R^2$ | Isomer* | Physical property | |
|---|---|---|---|---|---|
| 26 | $C_2H_5$- | Ph- | B | mp | 153-3.5°C |
| 27 | $C_2H_5$- | p-Cl-Ph- | M | $n_D^{25}$ | 1.5617 |
| 28 | $C_2H_5$- | $CH_3O$- | A | $n_D^{25}$ | 1.5333 |
| 29 | $C_2H_5$- | $CH_3O$- | B | mp | 98-9°C |
| 30 | n-$C_3H_7$- | $CH_3$ - | A | $n_D^{25}$ | 1.5304 |
| 31 | n-$C_3H_7$- | $CH_3$- | B | $n_D^{25}$ | 1.5264 |
| 32 | n-$C_3H_7$- | $C_2H_5$- | M | $n_D^{25}$ | 1.5283 |
| 33 | n-$C_3H_7$- | n-$C_3H_7$- | M | $n_D^{25}$ | 1.5305 |
| 34 | n-$C_3H_7$- | i-$C_3H_7$- | M | $n_D^{25}$ | 1.5288 |
| 35 | n-$C_3H_7$- | $CH_3CH{=}CH$- | M | $n_D^{25}$ | 1.5433 |
| 36 | n-$C_3H_7$- | $ClCH_2CH_2CH_2$- | M | $n_D^{25}$ | 1.5332 |
| 37 | n-$C_3H_7$- | n-$C_4H_9$- | M | $n_D^{25}$ | 1.5265 |
| 38 | n-$C_3H_7$- | n-$C_5H_{11}$- | M | $n_D^{25}$ | 1.5202 |
| 39 | n-$C_3H_7$- | $(CH_3)_3CCH_2$- | M | $n_D^{25}$ | 1.5192 |
| 40 | n-$C_3H_7$- | n-$C_6H_{13}$- | M | $n_D^{25}$ | 1.5175 |
| 41 | $CH_2{=}CHCH_2$- | $CH_3$- | M | $n_D^{25}$ | 1.5395 |
| 42 | $CH_2{=}CHCH_2$- | n-$C_5H_{11}$- | M | $n_D^{25}$ | 1.5232 |
| 43 | $CH{\equiv}CCH_2$- | n-$C_5H_{11}$- | M | | semi-solid |
| 44 | n-$C_4H_9$- | $CH_3$- | M | $n_D^{25}$ | 1.5287 |
| 45 | n-$C_4H_9$- | $C_2H_5$- | M | $n_D^{25}$ | 1.5249 |
| 46 | n-$C_4H_9$- | $CH_3CCl_2$- | M | $n_D^{25}$ | 1.5337 |

| Compound No. | $R^1$ | $R^2$ | Isomer* | Physical property | |
|---|---|---|---|---|---|
| 47 | $n-C_4H_9-$ | $n-C_3H_7-$ | M | $n_D^{25}$ | 1.5222 |
| 48 | $n-C_4H_9-$ | $CH_2=CHCH_2-$ | M | | amorphous |
| 49 | $n-C_4H_9-$ | $n-C_4H_9-$ | M | $n_D^{25}$ | 1.5218 |
| 50 | $n-C_4H_9-$ | $(CH_3)_2CHCH_2-$ | A | $n_D^{25}$ | 1.5212 |
| 51 | $n-C_4H_9-$ | $(CH_3)_2CHCH_2-$ | B | mp | 90.5-1.5°C |
| 52 | $n-C_4H_9-$ | $n-C_5H_{11}-$ | M | $n_D^{25}$ | 1.5175 |
| 53 | $n-C_4H_9-$ | $(CH_3)_3CCH_2-$ | M | | semi-solid |
| 54 | $n-C_4H_9-$ | $n-C_4H_9CHBr-$ | M | $n_D^{25}$ | 1.5293 |
| 55 | $n-C_4H_9-$ | $n-C_6H_{13}-$ | M | $n_D^{25}$ | 1.5167 |
| 56 | $n-C_4H_9-$ | cyc-$C_6H_{11}-$ | M | | amorphous |
| 57 | $n-C_4H_9-$ | $n-C_8H_{17}-$ | M | $n_D^{25}$ | 1.5125 |
| 58 | $n-C_4H_9-$ | $n-C_{10}H_{21}-$ | M | $n_D^{25}$ | 1.5072 |
| 59 | $n-C_4H_9-$ | p-Cl-Ph- | M | mp | 80-6°C |
| 60 | $CH_3CH=CHCH_2-$ | $n-C_5H_{11}-$ | M | $n_D^{25}$ | 1.5253 |
| 61 | $CH_2=C(CH_3)CH_2-$ | $n-C_5H_{11}-$ | M | $n_D^{25}$ | 1.5254 |
| 62 | $n-C_5H_{11}-$ | $n-C_5H_{11}-$ | A | $n_D^{25}$ | 1.5143 |
| 63 | $n-C_5H_{11}-$ | $n-C_5H_{11}-$ | B | $n_D^{25}$ | 1.5153 |
| 64 | $(CH_3)_2C=CHCH_2-$ | $n-C_5H_{11}-$ | M | $n_D^{25}$ | 1.5256 |
| 65 | $n-C_7H_{15}-$ | $n-C_5H_{11}-$ | A | $n_D^{25}$ | 1.4965 |
| 66 | $n-C_7H_{15}-$ | $n-C_5H_{11}-$ | B | $n_D^{25}$ | 1.5183 |

| Compound No. | $R^1$ | $R^2$ | Isomer* | Physical property | |
|---|---|---|---|---|---|
| 67 | $n-C_{10}H_{21}-$ | $n-C_5H_{11}-$ | A | $n_D^{25}$ | 1.5009 |
| 68 | $n-C_{10}H_{21}-$ | $n-C_5H_{11}-$ | B | $n_D^{25}$ | 1.4984 |

* M represents that the isomer is a diastereomer mixture, A and B indicate that the diastereomer is separated into each of the isomers.

** Ph means phenyl group.

Formulation Example 1:

Compound No. 14 (20 parts), diatomaceous earth (75 parts), and surface active agent mainly composed of alkylbenzene sulfonic acid (5 parts) were uniformly pulverized and mixed to obtain a wettable powder.

Formulation Example 2:

Compound No. 14 (40 parts), white carbon (10 parts), diatomaceous earth (47 parts) "Sorpol" 5039 (trade mark of Toho Kagaku Kogyo K.K.; surface active agent mainly composed of polyoxyethylenealkylaryl ether sulfonate) (3 parts) were uniformly pulverized and mixed to obtain a wettable powder.

Formulation Example 3:

Compound No. 30 (30 parts), "Sorpol" 3005X (trade mark of Toho Kagaku Kogyo K.K.; mixture of non -ionic surface active agent and anionic surface active agent) (15 parts), xylene (25 parts) and dimethylformamide (30 parts) were mixed and dissolved to obtain an emulsifiable concentrate.

Formulation Example 4:

Compound No. 52 (2 parts) and N,N-kaolinite

clay (made by Tsuchiya Kaolin Co.) (98 parts) were mixed and pulverized to obtain a dust.

Test Example 1: Test for protective effect against rice blast

To rice seedlings at 3 - 4 leaf stage (variety : Nihonbare) grown in each pot of 6 cm diameter, was applied a wettable powder containing the compound according to this invention shown in Table 3 as an active ingredient, that had been diluted with water to a predetermined concentration shown in Table 3, at a rate of 10 mℓ per one pot through foliar application. After air-drying the liquid chemical, spores of blast fungi (*Pyricularia oryzae*) cultured in an oat meal medium were spray -inoculated to the test plants, which were kept in a humid chamber at 27°C for 24 hours and then left in a water bath within a greenhouse for three days. The number of disease stigmata was counted to calculate the protective value according to the following equation. The results are shown in Table 3.

$$\text{Protective value (\%)} = \frac{\begin{array}{l}\text{(number of disease stigmata per leaf in not-treated lot)}\\ \text{- (number of lesions per leaf in treated lot)}\end{array}}{\text{(number of disease stigmata per leaf in not-treated lot)}} \times 100$$

For making the usefulness of this invention more definite, similar tests were carried out by using known compounds most analogous to the compounds of the present invention as the comparative chemical and the results are also shown in Table 3.

Table 3: Test result for the protective effect against rice blast

| Compound No. | Protective value (%) | |
| --- | --- | --- |
| | Active ingredient concentration at 200 ppm | Active ingredient concentration at 100 ppm |
| 13 | 100 | 72 |
| 14 | 100 | 100 |
| 15 | 94 | 88 |
| 16 | 100 | 98 |
| 17 | 94 | 93 |
| 18 | 95 | 95 |
| 23 | 99 | 78 |
| 29 | 100 | 76 |
| 30 | 92 | 0 |
| 31 | 100 | 86 |
| 32 | 100 | 91 |
| 33 | 100 | 93 |
| 34 | 100 | 82 |
| 35 | 98 | 97 |
| 36 | 98 | 85 |
| 37 | 99 | 88 |

| Compound No. | Protective value (%) | |
|---|---|---|
| | Active ingredient concentration at 200 ppm | Active ingredient concentration at 100 ppm |
| 38 | 100 | 90 |
| 39 | 95 | 34 |
| 40 | 96 | 96 |
| 41 | 88 | - |
| 42 | 98 | 77 |
| 43 | 98 | 90 |
| 44 | 90 | 64 |
| 45 | 100 | 89 |
| 46 | 99 | 41 |
| 47 | 97 | 81 |
| 48 | 100 | 38 |
| 49 | 100 | 43 |
| 50 | 100 | 59 |
| 51 | 90 | 42 |
| 52 | 100 | 91 |
| 53 | 94 | 39 |
| 54 | 94 | 24 |
| 55 | 100 | 0 |
| 57 | 98 | 0 |

| Compound No. | Protective value (%) | |
|---|---|---|
| | Active ingredient concentration at 200 ppm | Active ingredient concentration at 100 ppm |
| 60 | 100 | 51 |
| 61 | 100 | 80 |
| 63 | 97 | 58 |
| 64 | 96 | 61 |
| Comparative chemical 1 *1 | 0 | - |
| Comparative chemical 2 *2 | 0 | - |
| Comparative chemical 3 *3 | 39 | - |
| Comparative chemical 4 *4 | 86 | 12 |

*1 Comparative chemical 1

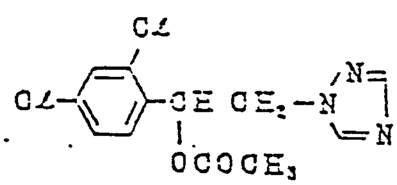

Compound (racemic modification) described in U.S. Patent No. 4349556.

*2 Comparative chemical 2

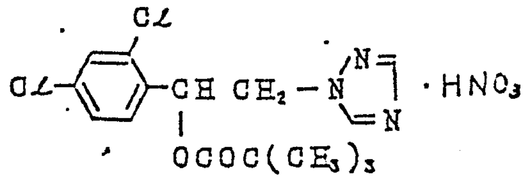

Compound (racemic modification) described in U.S. Patent No. 4349556.

*3 Comparative chemical 3

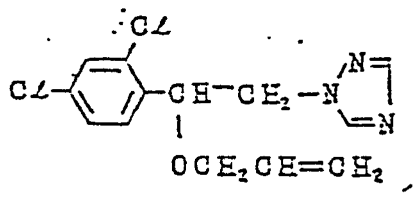

Compound (racemic modification) described in West German Patent Laid-Open No. 2556319.

*4 Comparative chemical 4

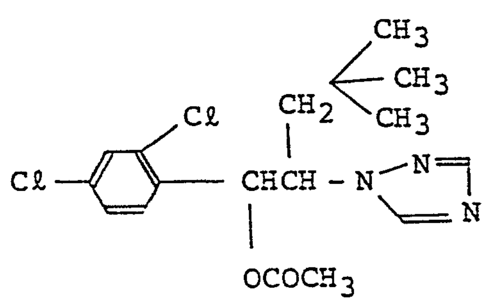

Compound described in West German Patent Laid-Open No. 3233145.

Test Example 2: Systemic activity test for rice blast

After washing the roots of rice seedlings at 3 - 4 leaf stage (variety : Nihonbare) grown in each pot of 6 cm diameter, the rice seedlings were transplanted in a hydroponic solution containing a wettable powder which contained the compound shown in Table 4 as the active ingredient and was prepared in the same manner as in Test Example 1 and diluted to a predetermined concentration shown in Table 4, and then left in a greenhouse for two days. After permeating the chemicals from the roots, spores of blast fungi (*Pyricularia oryzae*) cultivated in an oat meal medium were spray-inoculated to the test plants. Then after keeping in a humid chamber at 27°C for 24 hours, they were left in a greenhouse for three days and the protective value was calculated in the same manner as in Test Example 1.

The results are shown in Table 4.

Table 4: Results for the test of systemic activity against rice blast

| Compound No. | Active ingredient concentration (ppm) | Protective value (%) |
|---|---|---|
| 13 | 5 | 92 |
| 14 | 5 | 100 |
| 15 | 5 | 100 |
| 16 | 5 | 98 |
| 18 | 5 | 85 |
| 29 | 5 | 91 |
| 31 | 5 | 100 |
| 38 | 5 | 100 |
| 45 | 5 | 92 |

Test Example 3: Submerged application test against rice blast

Rice seedlings (variety : Nihonbare) were grown each by 7 hills per one pot made of synthetic resin of 11 cm diameter and the pot was put to submerged condition when the rice seedlings grew to 3 - 4 leaf stage. Then, the wettable powder containing the compound as shown in Table 5 as the active ingredient prepared in the same manner as in Test Example 1 was applied in a submerged manner by a predetermined amount shown in Table 5. 7 days after the application, spores of blast fungi (Pyricularia oryzae) cultivated in an oat meal medium were spray-inoculated to the test plants. Then they were treated in the same manner as in Test Example 1 and the protective value was calculated.

Further, the height of the rice plant was investigated seven days after the application and indicated in contrast with that in the non-treated lot.

The results are shown in Table 5.

Table 5: **Test results of the submerged application against rice blast**

| Compound No. | Active ingredient amount (kg/ha) | Protective value (Inoculation 7 days after application) (%) | Growth*6 control rate (%) |
|---|---|---|---|
| 14 | 1.2 | 90 | 87.0 |
| 31 | 1.2 | 88 | 88.7 |
| 32 | 1.2 | 92 | 84.1 |
| 33 | 1.2 | 85 | 90.6 |
| 34 | 1.2 | 92 | 87.1 |
| 35 | 1.2 | 86 | 90.8 |
| 37 | 1.2 | 98 | 85.1 |
| 38 | 1.2 | 78 | 87.9 |
| 40 | 1.2 | 90 | 87.5 |
| 45 | 1.2 | 58 | 88.6 |
| 52 | 1.2 | 90 | 89.6 |
| Comparative chemical 4 | 1.2 | 43 | — |
| Comparative chemical 5 *5 | 2.4 | 75 | 94.6 |
| Not-treated | — | — | 100.0 |

*5 Propenazole

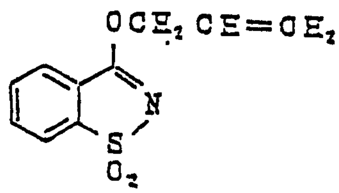

*6

$$\text{Growth control rate (\%)} = \frac{\text{hight of plant in treated lot}}{\text{hight of plant in non-treated lot}}$$

Test Example 4: Test for protective effect against cucumber grey mold

To cucumber seedlings at cotyledon-stage (variety : Chikanari-suyo) grown in each pot of 6 cm diameter, was applied the wettable powder containing the compound shown in Table 6 as the active ingredient which was prepared in the same manner as in Test Example 1 and diluted with water to a predetermined concentration shown in Table 6, at a ratio of 10 mℓ per one pot through foliar application. After air-drying the liquid chemical, grey mold fungi (Botrytis cinerea) cultured under shaking in an yeast glucose liquid medium were spray-inoculated to the test plants. Then, after keeping the test plants for four days in a humid chamber at 23°C, the state of the development of disease was investigated. The method of investigation was according to the following procedures. The disease rate was determined by at first examining the ratio of the disease area in the investigated leaves, classifying into indexes of 0, 1, 3, 5 depending on the degree of the ratio, investigating the number of leaves n0, n1, n3, n5 corresponding to each of the disease indexes and then calculating the disease ratio by the equation below (n is the total number of leaves investigated).

| Disease index | Ratio of disease area |
|---|---|
| 0 | none |
| 1 | about 1/4 of the entire leaf area |
| 3 | about 1/4 - 1/2 of the entire leaf area |
| 5 | more than 1/2 of the leaf entire area |

$$\text{Disease ratio} = \frac{0 \times n_0 + 1 \times n_1 + 3 \times n_3 + 5 \times n_5}{n}$$

The protective value was calculated by the following equation.

$$\text{Protective value (\%)} = \frac{\text{(disease ratio in the not-treated lot)} - \text{(disease ratio in the treated lot)}}{\text{(disease ratio in the not-treated lot)}} \times 100$$

The results are shown in Table 6.

Table 6: Test results for the protective effect against cucumber grey mold

| Compound No. | Active ingredient concentration (ppm) | Protective value (%) |
|---|---|---|
| 19 | 500 | 90 |
| 32 | 500 | 100 |
| 33 | 500 | 100 |
| 34 | 500 | 100 |
| 44 | 500 | 88 |
| 48 | 500 | 91 |
| 49 | 500 | 90 |
| 60 | 500 | 88 |

Test Example 5: Test for protective effect against wheat brown rust

To wheat seedlings at 1 - 2 leaf stage (variety : Norin-61) grown in each pot of 6 cm diameter, was applied the wettable powder containing the compound listed in Table 7 as the active ingredient which was prepared in the same manner as in Test Example 1 and diluted with water to a predetermined concentration shown in Table 7, at a rate of 10 mℓ per one pot through foliar application.

After air-drying the liquid chemical, suspension of spores obtained by grinding wheat suffered from wheat brown rust fungi (Puccinia recondita) was spray-inoculated to the test samples. Then, after keeping the test plants in a humid chamber at 22°C for 15 hours, they were left in a water bath within a greenhouse for 7 days.

The estimation was carried out by determining the ratio of the disease stigma area in each of the leaves and calculating the protective value according to the following equation.

The results are shown in Table 7.

$$\text{Protective value (\%)} = \frac{\text{(average ratio of disease stigma area in not-treated area)} - \text{(average ratio of disease stigma area in treated area)}}{\text{(average ratio of disease stigma area in not-treated area)}} \times 100$$

Table 7: Test results for the protective effect against wheat brown rust

| Compound No. | Active ingredient concentration (ppm) | Protective value (%) |
|---|---|---|
| 13 | 200 | 87 |
| 15 | 200 | 97 |
| 16 | 200 | 93 |
| 18 | 200 | 98 |
| 23 | 200 | 99 |
| 24 | 200 | 96 |
| 30 | 200 | 95 |
| 31 | 200 | 95 |
| 32 | 200 | 95 |
| 33 | 200 | 95 |
| 34 | 200 | 89 |

| Compound No. | Active ingredient concentration (ppm) | Protective value (%) |
|---|---|---|
| 36 | 200 | 96 |
| 38 | 200 | 98 |
| 39 | 200 | 92 |
| 40 | 200 | 86 |
| 44 | 200 | 96 |
| 45 | 200 | 98 |
| 46 | 200 | 98 |
| 47 | 200 | 98 |
| 49 | 200 | 98 |
| 50 | 200 | 96 |
| 52 | 200 | 96 |
| 55 | 200 | 97 |
| 57 | 200 | 84 |
| 60 | 200 | 94 |
| 61 | 200 | 98 |
| 62 | 200 | 91 |

Test Example 6: Test for protective effect against cucumber powdery mildew

To cucumber seedlings at cotyledon-stage (variety : Chikanari-suyo) grown in each pot of 6 cm diameter, was applied the wettable powder containing the compound shown in Table 8 as the active ingredient which was prepared in the same manner as in Test Example 1 and diluted with water to a predetermined concentration shown in Table 8, at a ratio of 10 mℓ per pot through foliar application.

After air-drying the liquid chemical, suspension of stores obtained by grinding cucumber leaves suffered from cucumber powdery mildew fungi (Sphaerotheca fuliginea) was spray-inoculated to the test plants and then they were left in a greenhouse from 7 to 10 days.

The estimation was carried out by determining the ratio of disease area in each of cotyledons and calculating the protective value by the following equation.

The results are shown in Table 8.

$$\text{Protective value (\%)} = \frac{\text{(average ratio of disease area in not-treated area)} - \text{(average ratio of disease area in treated area)}}{\text{(average ratio of disease area in not-treated area)}} \times 100$$

Table 8: Test results for the protective effect against cucumber powdery mildew

| Compound No. | Protective value (%) | |
|---|---|---|
| | Active ingredient concentration at 200 ppm | Active ingredient concentration at 50 ppm |
| 13 | 100 | 50 |
| 14 | 100 | 100 |
| 15 | 100 | 90 |
| 16 | 100 | 97 |
| 17 | 100 | 10 |
| 18 | 100 | 33 |
| 19 | 100 | 100 |
| 20 | 100 | 100 |
| 21 | 100 | 99 |
| 22 | 100 | 100 |
| 23 | 100 | 100 |
| 24 | 100 | 100 |
| 25 | 100 | 90 |
| 26 | 100 | 88 |
| 27 | 100 | 100 |
| 28 | 100 | 0 |
| 29 | 100 | 75 |

| Compound No. | Protective value (%) | |
|---|---|---|
| | Active ingredient concentration at 200 ppm | Active ingredient concentration at 50 ppm |
| 30 | 100 | 57 |
| 31 | 100 | 100 |
| 32 | 100 | 100 |
| 33 | 100 | 100 |
| 34 | 100 | 100 |
| 35 | 100 | 93 |
| 36 | 100 | 100 |
| 37 | 100 | 100 |
| 38 | 100 | 100 |
| 39 | 100 | 100 |
| 40 | 100 | 100 |
| 41 | 100 | 87 |
| 42 | 100 | 100 |
| 43 | 100 | 100 |
| 44 | 100 | 100 |
| 45 | 100 | 100 |
| 46 | 100 | 100 |
| 47 | 100 | 100 |
| 48 | 100 | 100 |

| Compound No. | Protective value (%) | |
|---|---|---|
| | Active ingredient concentration at 200 ppm | Active ingredient concentration at 50 ppm |
| 49 | 100 | 100 |
| 50 | 100 | 100 |
| 51 | 100 | 100 |
| 52 | 100 | 100 |
| 53 | 100 | 91 |
| 54 | 100 | 100 |
| 55 | 100 | 100 |
| 56 | 100 | 100 |
| 57 | 100 | 100 |
| 58 | 100 | 100 |
| 59 | 100 | 98 |
| 60 | 100 | 93 |
| 61 | 100 | 100 |
| 62 | 100 | 100 |
| 63 | 100 | 100 |
| 64 | 100 | 98 |
| 65 | 100 | 95 |
| 66 | 100 | 95 |
| 67 | 100 | 100 |
| 68 | 100 | 98 |

WHAT IS CLAIMED IS:

1. Triazolylacyloxy ethanes represented by the following general formula:

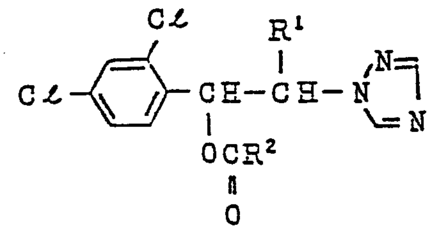

where $R^1$ represents linear alkyl; linear or branched alkenyl; or linear or branched alkynyl; $R^2$ represents linear or branched alkyl optionally substituted with halogen; linear or branched alkenyl; cycloalkyl; phenyl optionally substituted with lower alkyl or halogen; or lower alkoxy.

2. The compound as defined in Claim 1, wherein $R^1$ represents $C_1$ - $C_{12}$ linear alkyl; $C_2$ - $C_6$ linear or branched alkenyl; or $C_2$ - $C_6$ linear or branched alkynyl; and $R^2$ represents $C_1$ - $C_{12}$ linear or branched alkyl optionally substituted with fluorine, chlorine or bromine; $C_2$ - $C_6$ linear or branched alkenyl; $C_3$ - $C_6$ cycloalkyl; phenyl optionally substituted with $C_1$ - $C_3$ alkyl group, fluorine, chlorine or bromine; or $C_1$ - $C_4$ alkoxy.

3. The compound as defined in Claim 2, wherein $R^2$ represents $C_1$ - $C_{10}$ linear or branched alkyl optionally substituted with fluorine, chlorine or bromine; or $C_2$ - $C_6$ linear or branched alkenyl.

4. The compound as defined in Claim 2, wherein $R^1$ represents $C_1$ - $C_{10}$ linear alkyl; $C_2$ - $C_5$ linear or branched alkenyl; or $C_2$ - $C_4$ linear alkynyl and $R^2$ represents $C_1$ - $C_{10}$ linear or branched alkyl optionally substituted with fluorine, chlorine or bromine; or $C_2$ - $C_6$ linear or branched alkenyl.

5. The compound as defined in Claim 4, wherein $R^1$ represents $C_1$ - $C_{10}$ linear alkyl; $C_3$ - $C_5$ linear or branched alkenyl; or propargyl and $R^2$ represents $C_1$ - $C_{10}$ linear or branched alkyl optionally substituted with fluorine, chlorine or bromine; or $C_2$ - $C_3$ alkenyl.

6. The compound as defined in Claim 5, wherein $R^2$ represents $C_1$ - $C_8$ linear alkyl optionally substituted with fluorine, chlorine or bromine.

7. The compound as defined in Claim 5,

wherein $R^1$ represents $C_1 - C_8$ linear alkyl and $R^2$ represents $C_1 - C_8$ linear alkyl optionally substituted with fluorine, chlorine or bromine.

8. The compound as defined in Claim 7, wherein $R^1$ represents $C_1 - C_6$ linear alkyl and $R^2$ represents $C_1 - C_8$ linear alkyl optionally substituted with chlorine or bromine.

9. The compound as dedined in Claim 8, wherein $R^1$ represents $C_2 - C_4$ linear alkyl and $R^2$ represents $C_1 - C_6$ linear alkyl optionally substituted with chlorine or bromine.

10. A fungicidal composition containing a triazolylacyloxy ethane according to any of Claims 1 to 9 as the active ingredient.

11. The fungicidal composition as defined in Claim 10 comprising from 0.1 to 80% by weight of triazolylacyloxy ethanes as the active ingredient and from 20 to 99.9% by weight of an inert other component.

12. A method of controlling fungi, wherein an effective amount of a triazolylacyloxy ethane according to any of Claims 1 to 9 is applied to plants or a habitat thereof.

European Patent Office

# EUROPEAN SEARCH REPORT

0173807 Application number

EP 85 10 7718

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | DE-A-2 640 823 (IMPERIAL CHEMICAL INDUSTRIES LTD.) * Page 1, line 1 - page 3, line 6, in particular page 2, lines 10-12; page 9, line 25 - page 12, line 37 * & US-A-4 315 016 (Cat. D) | 1-12 | C 07 D 249/08<br>A 01 N 43/653 |
| Y | DE-A-2 628 419 (BAYER AG) * Page 5, lines 1-19; page 19, line 1 - page 22, line 28 * & US-A-4 349 556 (Cat. D) | 1-12 | |
| Y | EP-A-0 102 559 (BASF AG) * Page 1, line 30 - page 2, line 27; page 14, lines 1-37; page 15, line 36 - page 17, line 6 * & DE-A-3 233 145 (Cat. D) | 1-12 | |
| A | EP-A-0 010 287 (BASF AG) * Page 2, line 4 - page 3, line 13 * | 1 | TECHNICAL FIELDS SEARCHED (Int. Cl.4)<br>C 07 D 249/00<br>C 07 D 521/00<br>A 01 N 43/00 |
| A | EP-A-0 019 762 (BASF AG) * Page 1, line 32 - page 2, line 25 * | 1 | |

The present search report has been drawn up for all claims

| Place of search | Date of completion of the search | Examiner |
|---|---|---|
| BERLIN | 26-08-1985 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82